**Europäisches Patentamt**

**European Patent Office**

**Office européen des brevets**

(11) Veröffentlichungsnummer: **0 259 751**
**A2**

(12) # EUROPÄISCHE PATENTANMELDUNG

(21) Anmeldenummer: **87112717.1**

(22) Anmeldetag: **01.09.87**

(51) Int. Cl.⁴: **C07G 11/00** , **C12P 1/04** , **A61K 31/71**

Der (Die) Mikroorganismus (Mikroorganismen) ist (sind) bei Deutsche Sammlung von Mikroorganismen unter der (den) Nummer(n) DSM 3817 hinterlegt worden.

Die Bezeichnung der Erfindung wurde geändert (Richtlinien für die Prüfung im EPA, A-III, 7.3).

(30) Priorität: **12.09.86 DE 3631008**

(43) Veröffentlichungstag der Anmeldung:
**16.03.88 Patentblatt 88/11**

(84) Benannte Vertragsstaaten:
**AT BE CH DE FR GB IT LI NL**

(71) Anmelder: **BAYER AG**
**Konzernverwaltung RP Patentabteilung**
**D-5090 Leverkusen 1 Bayerwerk(DE)**

(72) Erfinder: **Bischoff, Erwin, Dr.**
**Pahlkestrasse 73**
**D-5600 Wuppertal 1(DE)**
Erfinder: **Müller, Hartwig, Dr.**
**Steinstrasse 15**
**D-5620 Velbert 15(DE)**
Erfinder: **Salcher, Olga, Dr.**
**Am Eckbusch 43/100**
**D-5600 Wuppertal 1(DE)**
Erfinder: **Berschauer, Friedrich, Dr.**
**Claudiusweg 9**
**D-5600 Wuppertal 1(DE)**
Erfinder: **Scheer, Martin, Dr.**
**Herberts-Katernberg 7**
**D-5600 Wuppertal 1(DE)**
Erfinder: **De Jong, Anno, Dr.**
**Stockmannsmühle 46**
**D-5600 Wuppertal 1(DE)**
Erfinder: **Frobel, Klaus Dr.**
**Birkenhöhe 5**
**D-5600 Wuppertal 1(DE)**

(54) **Annomycin, ein Antibiotikum; mikrobiologisches Verfahren zu dessen Herstellung und seine Verwendung.**

(57) Die vorliegende Erfindung betrifft die neue organischchemische Verbindung Annomycin die antibakterielle Wirkung aufweist und sich als Leistungsförderer für Tiere einsetzen läßt.

EP 0 259 751 A2

## Organisch-chemische Verbindung, mikrobiologische Verfahren zu ihrer Herstellung sowie ihre Verwendung

Die vorliegende Erfindung betrifft eine neue organisch-chemische Verbindung, mikrobiologische Verfahren zu ihrer Herstellung sowie ihre Verwendung als Mittel zur Leistungsförderung bei Tieren.

Die erfindungsgemäße Verbindung läßt sich durch die folgenden Eigenschaften und Parameter charakterisieren:

1. IR-KBr-Absorptionsspektrum

Es zeigt, bei folgenden Wellenlängen (ausgedrückt in $cm^{-1}$) charakteristische Absorptionsbanden:

| | |
|------|------|
| 3388 | 1409 |
| 2936 | 1355 |
| 1731 | 1188 |
| 1639 | 1073 |
| 1536 | 972 |
| | 811 |
| | 652 |

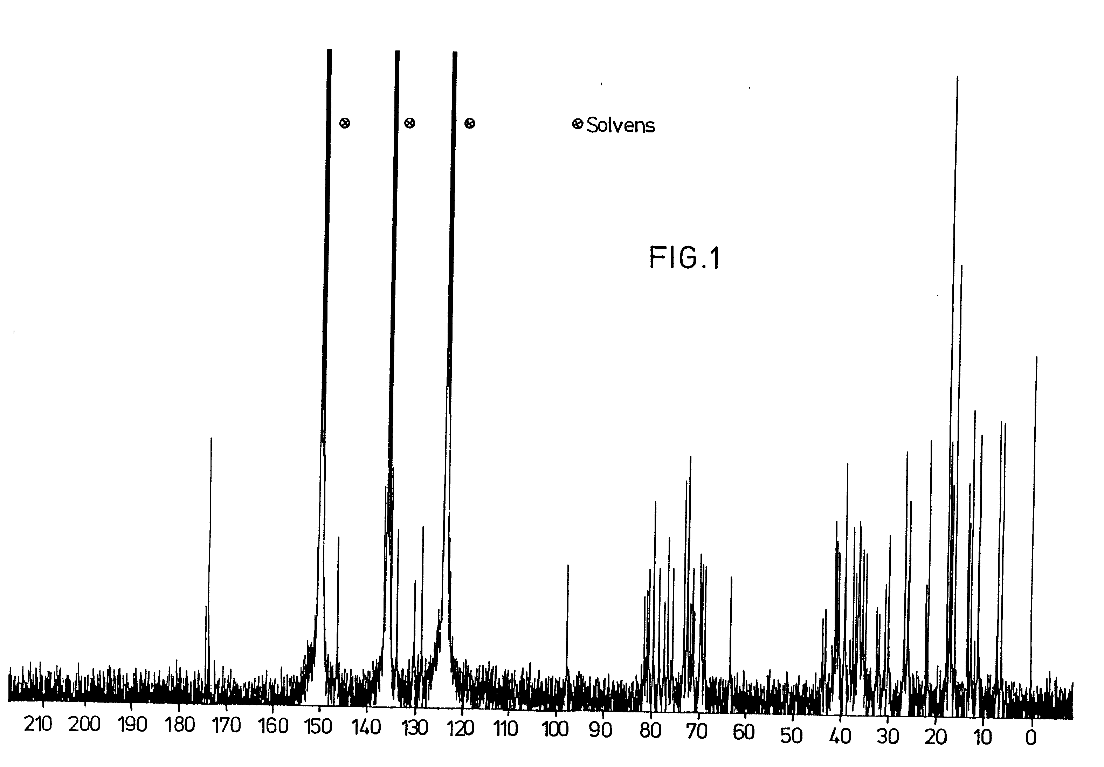

2. 13C-Kernresonanzspektrum des Antibiotikums in Pyridin, gemäß Abb. 1, angegeben in Teilen pro Million.

Es wurde mit einer Feldstärke von 75,48 MHz an einer Lösung des Antibiotikums in deuteriertem Pyridin mit Tetramethylsilan als innerem Standard aufgenommen.

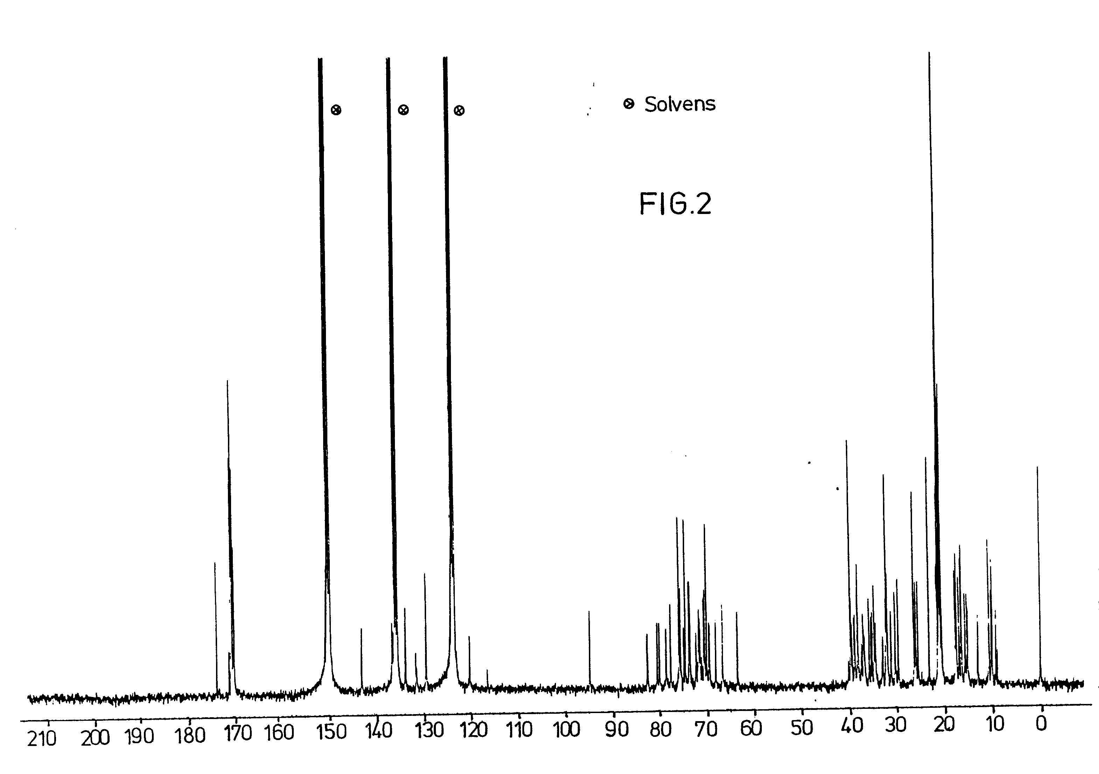

3. Das 13C-Kernresonanzspektrum des mit Acetanhydrid umgesetzten Antibiotikums in deuteriertem Pyridin gemäß Abb. 2, angegeben in Teilen pro Million.

Es wurde mit einer Feldstärke von 75,48 MHz an einer Lösung des Acetates in deuteriertem Pyridin mit Tetramethylsilan als innerem Standard aufgenommen.

4. Elementaranalyse (nach 2 Tagen Trocknen im Hochvakuum bei 30°C):

| | |
|---|------------|
| C | 57,4 - 56,8 |
| H | - 8,6 - 8,8 |
| N | 3,3 - 3,4 |
| O | 26,1 - 28,2 |

Es muß hier darauf hingewiesen werden, daß bei hochmolekularen Naturstoffen die Fehlerbreite der Elementaranalyse größer sein kann als allgemein üblich; daher ist eine genaue Bestimmung der Summenformel nicht möglich (R.B. Woodward, Angew. Chem. 69, 50-51 (1957)).

5. Die Verbindung läßt sich nach Chromatographie auf Kieselgel-Dünnschichtplatten mit Thymol/$H_2SO_4$ oder $Cl_2$-Tolidin Reagenz anfärben. Die Reagenzien wurden nach den üblichen Vorschriften (vgl. E. Stahl, Dünnschichtchromatographie, 2. Aufl., Springer Verlag, Berlin, Heidelberg, New York 1967) hergestellt.

6. Die antibakterielle Wirkung der Verbindung ist in Tabelle 1 wiedergegeben.

Das neue erfindungsgemäße Antibiotikum erhält die Bezeichnung Annomycin.

Des weiteren betrifft die vorliegende Erfindung neue Mikroorganismen der Familie Streptomycetaceae, die bei der Kultivierung in einem Kohlenstoff-und Stickstoffquellen sowie Mineralsalze enthaltenden Nährmedium eine Verbindung produzieren, welche die oben unter (1) bis (5) aufgeführten Eigenschaften und Parameter aufweist.

Von diesen ist der neue Mikroorganismenstamm BS 572 der Gattung Streptomyces im Rahmen der vorliegenden Erfindung besonders wichtig.

Die Anreicherung und Isolierung des Stammes erfolgte nach den üblichen Methoden der Actinomyceten-Isolierung durch Ausplattieren von Erdprobensuspensionen auf Petrischalen, vier-bis sechswöchige Bebrütung und wiederholter Abimpfung von Einzelkolonien (Williams, S.T. and Cross, T. (1971), Actinomycetes, in Methods in Microbiology 4, 295-334. Booth, C. (editor), London-New York: Academic Press).

Der neue Streptomyces-Stamm mit der Laborbezeichnung BS 572 wurde am 13.08.86 unter der Nummer DSM 3817 bei der Deutschen Sammlung von Mikroorganismen, Grisebachstraße 8, 3400 Göttingen, BRD, hinterlegt.

Es wurde weiterhin gefunden, daß man die erfindungsgemäße Verbindung erhält, wenn man geeignete Mikroorganismen der Familie Streptomycetaceae in einem Nährmedium, welches assimilierbare Kohlenstoff-und Stickstoffquellen sowie Mineralsalze enthält, unter aeroben Bedingungen kultiviert und die Verbindung nach üblichen Methoden isoliert.

Zur Durchführung des erfindungsgemäßen Verfahrens kann insbesondere der Streptomyceten-Stamm BS 572 (sowie seine Mutanten und Varianten) Verwendung finden.

Dieser Stamm gehört zu den Bakterien der Ordnung Actinomycetales, der Familie Streptomycetaceae und der Gattung Streptomyces an. Er wurde aus Erde isoliert.

Die taxonomische Bestimmung des Stammes BS 572 erfolgte nach Bergey's Manual of Determinative Bacteriology 8 th ed. (1974) sowie International Journal of Systematic Bacteriology 16, 313-340 (1966) und The Prokaryotes 2, 2028-2090 (1981).

## 1, Morphologie

Eine mäßige Sporulation war nur auf Basalagar mit Raffinose und Galactose (ISP-Medium Nr. 9) und auf ISP-Medium Nr. 3 + 7 zu beobachten. Auf anderen Medien (ISP-Medien Nr. 1 + 2 und 4-6) wurde sehr wenig Luftmyzel bzw. nur Substratmyzel gebildet.

### Luftmyzel (ISP-Medium Nr. 9; 28°C; 7 d):

Farbe: nach 7d blaßrosa
Sporenketten: RF-Typ → engl.: Spore chain morphology: Rectus flexibilis
Sporen: glatt (Elektronenmikroskopie), zylindrisch ca. 1,2-1,4 μm breit, 1,8-2,4 μm lang

### Substratmyzel:

Farbe: hellbraun bis rotbraun

## 2. Physiologie

Das Temperaturoptimum liegt bei 28°C (ISP-Medium Nr. 2, 2d). Das Wachstum wird durch Chloramphenicol (10 μg); Erythromycin (10 μg), Sulphafurazol (100 μg), Streptomycin (10 μg), Tetracyclin (10 μg), Fusidinsäure (10 μg), Novobiocin (5 μg) gehemmt (ISP-Medium Nr. 2, 28°C, 2d). Gutes Wachstum (Substratmyzel) erfolgt auf den ISP-Medien Nr. 3 + 7 spärlicher Luftmyzelbildung auf ISP-Medium Nr. 3.

Im folgenden wird die Zusammensetzung der oben angegebenen Medien in denen Sporulation von BS 572 erfolgte beschrieben:

-ISP-Medium Nr. 7 (Tyrosin agar)Glycerol          15 g

| | |
|---|---|
| L-Tyrosin | 0,5 g |
| L-Asparagin | 1 g |
| $K_2HPO_4$ (anhydr.) | 0,5 g |
| $MgSO_4 \bullet 7H_2O$ | 0,5 g |
| NaCl | 0,5 g |
| $FeSO_4 \bullet 7H_2O$ | 0,01 g |
| $H_2O$ deionisiert | 1000 ml |
| Spurenelementelösung | 1 ml |
| pH 7,2 - 7,4 | |
| Bacto-Agar | 20 g |

Spurenelementlösung $FeSO_4 \bullet 7H_2O$          0,1 g

| | |
|---|---|
| $MnCl_2 \bullet 4H_2O$ | 0,1 g |
| $ZnSO_4 \bullet 7H_2O$ | 0,1 g |
| $H_2O$ deionisiert | 100 ml |

-Basalagar mit Raffinose oder Galactose(NH₄)₂SO₄          2,64 g
KH₂PO₄ (anhydrous)          2,38 g
K₂HPO₄ •3H₂O          5,65 g
MgSO₄ • 7H₂O          1,00 g
Pridham and Gottlieb Spurenelementlösung (B)          1000 ml
H₂O deionisiert          1000 ml
pH 6,8 - 7,0
Difco Agar          15 g
Raffinose o. Galactose 10 g*

   \* Nach dem Autoklavieren werden steril filtrierte Lösungen von Raffinose oder Galactose zugesetzt. Endkonzentration 10 g pro l.


Pridham and Gottlieg Spurenelementlösung (B)CuSO₄ • 5H₂O          0,64 g
FeSO₄ • 7H₂O          0,11 g
MnCl₂ • 4H₂O          0,79 g
ZnSO₄ • 7H₂O          0,15 g
H₂O deionisiert          100 ml

   Weitere medien siehe Int. J. Syst. Bact 16, 313-340 (1966).
   Die Verwertung von C-Quellen wurde auf Basalagar nach Int. I. Syst. Bact. 16, 313-240 (1966) überprüft.
Für die Negativ-Kontrolle wurde das Wachstum auf Basalagar ohne C-Quelle verglichen.

| C-Quelle (10 g/l) | Wachstum* |
|---|---|
| Kontrolle (keine C-Quelle | - |
| D-Glucose | ± |
| D-Xylose | ± |
| D-Arabinose | - |
| L-Rhamnose | + |
| D-Fructose | - |
| D-Galactose | + |
| Raffinose | + |
| D-Mannit | + |
| Meso-Inosit | - |
| Salicin | - |
| Saccharose | - |
| Ribose | + |
| Mannose | + |
| Maltose | - |
| Lactose | - |
| Melibiose | - |
| Cellulose | - |
| Acetat | - |

\*) + = Wachstum;

    - = kein Wachstum;

    ± = Ergebnis nicht eindeutig

Der Stamm BS 572 isoliert aus einer Erdprobe aus Israel (Jerusalem), läßt sich aufgrund der morphologischen Daten in die Cinnamomeus-Gruppe der Streptomyceten einordnen. Die physiologischen Eigenschaften stimmen mit keinem der in Bergey's Manual beschriebenen Stämme völlig überein. Taxonomische Bezeichnung: Streptomyces sp.

Für das Verfahren zur Herstellung der erfindungsgemäßen Verbindung verwendet man Nährmedien, die die üblichen Kohlenstoff-und Stickstoffquellen und die notwendigen Salze enthalten. Als Kohlenstoffquelle können verwendet werden:

Kohlenhydrate, insbesondere Polysaccharide, wie. z.B. Stärke, Oligosaccharide, wie z.B. Raffinose und Monosaccharide, wie z.B. Mannose und Galactose. Weiterhin sind auch Zuckeralkohole, wie z.B. Mannit und Glyzerin, als Kohlenhydratquelle geeignet. Außerdem können auch natürlich vorkommende Gemische, wie z.B. Malzextrakt oder Distiller soluble, sowie Gemische aus den erwähnten Möglichkeiten verwendet werden.

Als N-Quelle können die üblichen stickstoffhaltigen Nährmedienbestandteile Verwendung finden, so z.B. Aminosäuren, Eiweißstoffe, Eiweißhydrolysate, Ammoniumsalze, natürlich vorkommende komplexe Stoffe, wie Sojabohnenmehl, Milchpulver und geeignete Mischungen derselben.

Als Hilfsstoffe werden im Nährmedium Mineralsalze benötigt, z.B. Phosphate, Sulfate oder Chloride, des Kaliums, des Natriums, des Calciums, des Magnesiums, Eisens, Zinks und Mangans. Die Konzentration dieser Stoffe kann in weiten Grenzen schwanken, z.T. sind die notwendigen Konzentrationen der Mineralsalze in den o.a. Kohlenstoff-oder Stickstoffquellen oder im verwendeten Wasser als Verunreinigungen enthalten.

Weiterhin können als Hilfsstoffe noch Antischaummittel der verschiedensten Art Verwendung finden, wie z.B. Polyole oder Silikone.

Das Verfahren zur Herstellung der erfindungsgemäßen Verbindung kann mit Hilfe üblicher fester, halbfester oder flüssiger Nährmedien durchgeführt werden. Bevorzugt werden wäßrig flüssige Nährmedien.

Die Beimpfung der Nährmedien erfolgt dabei nach allgemein üblichen Methoden, z.B. über Schrägröhrchen oder Kolbenkulturen.

Die Kultur erfolgt unter aeroben Bedingungen und kann gemäß den allgemein üblichen Methoden wie unter Verwendung von Schüttelkulturen z.B. in Schüttelkolben, von luftbewegten Kulturen oder von Submerskulturen durchgeführt werden. Bevorzugt erfolgt die Kultivierung in aeroben Submersverfahren in belüfteten Fermentern, z.B. in üblichen Submersfermentationstanks. Es ist möglich, die Kultur kontinuierlich oder diskontinuierlich durchzuführen. Vorzugsweise wird diskontinuierlich gearbeitet.

Bei der Durchführung des Herstellungsverfahrens wird unter aeroben Bedingungen gearbeitet; die Kultur kann gemäß üblicher Methoden, so z.B. unter Verwendung von Schüttelkulturen oder von belüfteten Fermenterkulturen, durchgeführt werden. Die Prozentverhältnisse der Nährlösungsbestandteile können in weiten Bereichen schwanken, im allgemeinen machen die Kohlenstoffquellen 0,5 bis 8 %, vorzugsweise 0,6 bis 6 % aus, die Stickstoffquellen 0,1 bis 5 %, vorzugsweise 0,5 bis 2 %, aus, die Salze liegen in üblichen Konzentrationen vor, vorzugsweise im Bereich zwischen 0,001 bis 0,5 Gew.-%. Die Antischaummittel liegen in bis zu 0,5 %iger Konzentration vor. Die zur Sterilisation angewandten Temperaturen liegen bei 100 bis 140°C, bevorzugt bei 120 bis 130°C.

Der pH-Wert der wachsenden Kulturen sollte vorzugsweise zwischen etwa 6 und etwa 8,5, insbesondere zwischen 6,5 und 8,0 gehalten werden. Ein zu starker pH-Abfall in den sauren Bereich kann durch Zusätze einer organischen oder anorganischen Base, vorzugsweise von $CaCO_3$ vermieden werden. Wie in der Fermentationstechnologie üblich, kann auch eine automatische pH-Regulierung durchgeführt werden, bei der sterile organische oder anorganische Säure, z.B. $H_2SO_4$, oder sterile Lauge, z.B. NaOH in Abständen in die Kulturlösung eingespritzt wird.

Es ist zweckmäßig sicherzustellen, daß die Mikroorganismen ausreichend mit Sauerstoff sowie den Nährstoffen in Kontakt gebracht werden. Dies kann nach den allgemein üblichen Methoden wie Schütteln und Rühren erfolgen.

Die Züchtungstemperatur kann zwischen etwa 16 und etwa 42°C, vorzugsweise zwischen 24 und 32°C liegen, besonders bevorzugt liegt sie bei etwa 28°C. Die Dauer der Züchtung kann stark variiert werden, wobei z.B. die Zusammensetzung des Nährmediums und die Züchtungstemperatur eine Rolle spielen. Die jeweiligen optimalen Bedingungen können von jedem Fachmann auf dem mikrobiologischen Gebiet leicht festgelegt werden.

Es hat sich herausgestellt, daß die Menge der sich in der Kulturbrühe anreichernden erfindungsgemäßen Verbindung im allgemeinen ihr Maximum etwa 1 bis 7, vorzugsweise 1 bis 4 Tage nach Züchtungsbeginn erreicht.

Wie allgemein bei mikrobiologischen Verfahren sollten Fremdinfektionen der Kulturmedien vermieden werden. Hierzu werden die üblichen Vorkehrungen getroffen, wie Sterilisation der Nährmedien, der Kulturgefäße sowie der für die Belüftung notwendigen Luft. Zur Sterilisation der Vorrichtung können z.B. die Dampf - oder Trockensterilisation verwendet werden.

Falls bei der Kultivierung in unerwünschter Menge Schaum entsteht, können übliche chemische Schaumdämpfungsmittel, z.B. flüssige Fette und Öle, Öl-Wasser-Emulsionen, Paraffine, höhere Alkohole, wie Octodecanol, Siliconöle, Polyoxyethylen-bzw. Polyoxypropylenverbindungen zugesetzt werden. Schaum kann auch mit Hilfe der üblichen mechanischen Vorrichtungen (welche z.B. Zentrifugalkräfte benutzen) gedämpft oder beseitigt werden.

Der erfindungsgemäße Wirkstoff zeigt antibiotische Wirksamkeit gegen grampositive Bakterien.

Die mikrobiologische Aktivität von Annomycin wird in Tabelle 1 beschrieben. Die Testmethode war die konventionelle Agardiffusionsmethode. In die vorgestanzten Löcher (Ø 9 mm) eines Nährbodens, der den angegebenen Titer des Testkeims enthält, werden 100 µl einer methanolischen Lösung des erfindungsgemäßen Wirkstoffs Annomycin in den angegebenen Konzentrationen gegeben. Anschließend werden die Agarplatten bei der angegebenen Temperatur inkubiert. Nach der angegebenen Inkubationszeit wird der Durchmesser des durch Annomycin hervorgerufenen Hemmhofes gemessen.

## Tabelle 1

Wirkungsspektrum von Annomycin

| Testkeim: | Hemmhofdurchmesser in mm bei Konzentration | | Nährboden | Titer | Temperatur | Inkubationszeit |
|---|---|---|---|---|---|---|
| | 0,2 mg/ml | 1 mg/ml | | | | |
| Arthrobacter oxidans DSM 20119 | 15 | 19 | St 1 | $1 \times 10^5$ | 28° C | 40 h |
| Arthrobacter simplex NCIB 9770 | 15 | 20 | St 1 | $1 \times 10^6$ | 28° C | 48 h |
| Bacillus brevis (Sporen) DSM 298 | 10 | 14 | A 3 | $1 \times 10^6$ | 37° C | 16 h |
| Bacillus cereus (Sporen) DSM 318 | 11 | 16 (24) | A 3 | $1 \times 10^6$ | 37° C | 16 h |
| Bacillus subtilis (Sporen) ATCC 6633 | 12 | 18 | A 3 | $1 \times 10^6$ | 37° C | 16 h |
| Bacillus subtilis (Sporen) ATCC 27859 | 25 | 31 | A 3 | $1 \times 10^6$ | 37° C | 16 h |
| Cornynebacterium facians DSM 20131 | 14 (23) | 20 (30) | St 1 | $1 \times 10^6$ | 28° C | 40 h |
| E. coli 14 | - | - | A 3 | $1 \times 10^6$ | 37° C | 16 h |
| E. coli A 261 | - | - | A 3 | $1 \times 10^6$ | 37° C | 16 h |
| E. coli ATCC 9637 | - | - | A 3 | $1 \times 10^6$ | 37° C | 16 h |
| E. coli ATCC 11105 | - | - | A 3 | $1 \times 10^6$ | 37° C | 16 h |
| E. coli ATCC 25290 | - | - | A 3 | $1 \times 10^6$ | 37° C | 16 h |
| Klebsiella pneumoniae DSM 30102 | - | - | A 3 | $1 \times 10^6$ | 37° C | 16 h |

0 259 751

**Tabelle 1** (Fortsetzung)

Wirkungsspektrum von Annomycin

| Testkeim: | Hemmhofdurchmesser in mm bei Konzentration | | Nährboden | Titer | Tempera-tur | Inkubations-zeit |
|---|---|---|---|---|---|---|
| | 0,2 mg/ml | 1 mg/ml | | | | |
| Micrococcus luteus | 23 | 28 | A 3 | $1 \times 10^6$ | 37°C | 16 h |
| Proteus vulgaris | - | - | A 3 | $1 \times 10^6$ | 37°C | 16 h |
| Pseudomonas aeruginosa | - | - | A 3 | $1 \times 10^6$ | 37°C | 16 h |
| Serratia marcescens | - | - | A 3 | $1 \times 10^6$ | 37°C | 16 h |
| Staphylococcus aureus 1756 | 12 | 20 | A 3 | $1 \times 10^6$ | 37°C | 16 h |
| Staphylococcus aureus P 209 | 13 | 21 | A 3 | $1 \times 10^6$ | 37°C | 16 h |
| Saccharomyces cerevisiae | - | - | YM | $1 \times 10^4$ | 28°C | 16 h |
| Piricularia orycae (Sporen) DSM 62938 | - | - | YM | $1 \times 10^4$ | 22°C | 20 h |

Nährboden:

St 1 = Standard-1-Nährbouillon (Merck 7882) + 15 g Bacto Agar pro Liter

YM = Yeast-Malt nach ATCC 196 + 15 g Bacto-Agar pro Liter

A 3 = Antibiotic Medium 3 (Difco 0243-01-6) + 15 g Bacto-Agar pro Liter

Die erfindungsgemäße Verbindung kann aus dem Kulturmedium nach allgemein üblichen physikalisch-chemischen Methoden isoliert werden. Die Isolierung kann z.B. gemäß den üblichen Extraktionsverfahren, Fällungsverfahren und/oder Chromatographieverfahren erfolgen. Die isolierte Verbindung kann mit Hilfe der genannten Methoden auch feingereinigt werden. Für viele Fälle ist jedoch eine Feinreinigung nicht erforderlich, da die gegebenenfalls vorhandenen Verunreinigungen die Wirksamkeit der Verbindung nicht nachteilig beeinflussen.

Um bei den o.a. Isolierungs-und Reinigungsmethoden die Fraktionen herauszufinden, in welchen die erfindungsgemäße Verbindung in höchster Konzentration bzw. Reinheit vorliegt, wird bevorzugt die Bestimmung der antibiotischen Aktivität herangezogen werden. Als Testkeime eignen sich dabei besonders Staphylococcus aureus 1756, Bac. subtilis ATCC 27859.

Die Isolierung und Reinigung der erfindungsgemäßen Verbindung kann z.B. im Falle, daß ein flüssiges wäßriges Nährmedium verwendet wird, wie folgt vorgenommen werden: Nach seiner Anreicherung im Kulturüberstand werden Kulturfiltrat und Mycel mit üblichen Methoden separiert (z.B. Zentrifugation).

Aus dem Kulturfiltrat kann die erfindungsgemäße Verbindung mit Hilfe von üblichen Extraktionsverfahren, Fällungsverfahren und/oder Chromatographieverfahren isoliert und gegebenenfalls gereinigt werden. Die Chromato graphie kann in Form der Säulenchromatographie durchgeführt werden. Als Adsorptionsmittel können die üblichen anorganischen oder organischen Adsorptionsmittel eingesetzt werden, wie z.B. Aluminiumoxid, Kieselgel, Magnesiumsilikat, Aktivkohle, Cellulose, Cellulosederivate, Kunstharze wie Polyamiden, z.B. acetyliertes Polyamid oder Dextrangele. Als Laufmittel können die verschiedensten Lösungsmittel oder Lösungsmittelgemische verwendet werden, in welchen die erfindungsgemäße Verbindung löslich ist. Bevorzugt wird Methanol oder ein Gemisch aus Butanol : Eisessig : $H_2O$ im Verhältnis 2:1:1 eingesetzt.

Bevorzugt werden zur Isolierung der erfindungsgemäßen Verbindung Chromatographie-Verfahren verwendet, z.B. unspezifische Adsorption an hydrophobe Sorbentien oder andererseits Geldiffusionschromatographie.

Für die kommerzielle Herstellung der erfindungsgemäßen Verbindung bevorzugt man die Gewinnung durch Adsorption und anschließende Desorption an einem hydrophoben Trägerharz (z.B. Lewapol®; ein hydrophobes Trägerharz der Bayer AG). Die Desorption kann z.B. mit kurzkettigen aliphatischen Alkoholen durchgeführt werden, bevorzugt Methanol oder Ethanol.

Eine derart vorgereinigte Fraktion kann wiederum mit den üblichen Methoden gereinigt werden. Bevorzugt kann hier die Geldiffusionschromatographie eingesetzt werden. Die Chromatographie an Sephadex LH-20® verläuft erfolgreich. Ein derartig gewonnenes Produkt ist im allgemeinen reiner als 40 %.

Die erfindungsgemäße Verbindung kann aus dem Myzelium isoliert werden. Dazu wird das Myzelium von der Kulturbrühe vorzugsweise durch Zentrifugation abgetrennt und mit einem, mit Wasser mischbaren Lösungsmittel mehrfach, vorzugsweise zweimal extrahiert. Als Lösungsmittel können niedere Alkylalkohole ($C_1$-$C_4$) wie Ethanol oder Isopropanol sowie Ketone wobei besonders bevorzugt Aceton ist, verwendet werden. Die wäßrig organische Lösung wird im Vakuum konzentriert, bis die organische Phase abdestilliert ist. Nun wird mit Wasser auf das entsprechende Volumen der Kulturbrühe verdünnt, gefriergetrocknet und die Aufarbeitung wie unten angegeben weitergeführt.

Die erfindungsgemäße Verbindung kann auch aus dem Kulturfiltrat durch Adsorption an Aktivkohle bzw. an geeignete Harze isoliert werden. Als besonders geeignete Methode erwies sich die Bindung der erfindungsgemäßen Verbindung an unspezifische Adsorberharze auf Polystyrolbasis (z.B. Amberlite XAD® der Fa. Roehm & Haas oder Lewapol OC 1031® der Fa. Bayer). Die Desorption der erfindungsgemäßen Verbindung wird fraktioniert, durch Mischungen aus Wasser und organischen Lösungsmitteln, insbesondere Wasser/Methanol vorgenommen. Die durch Test gegen Staphylococcus aureus 1756 ermittelten aktiven Fraktionen werden unter reduziertem Druck bis zur vollständigen Entfernung des organischen Lösungsmittels konzentriert und der Rückstand in ca. 1/50 des Volumens des Kulturfiltrates suspendiert und gefriergetrocknet.

Die Isolierung aus dem Kulturfiltrat ist bevorzugt. Das Lyophylisat wird mit Wasser extrahiert, zentrifugiert und der Rückstand erneut in Wasser suspendiert und gefriergetrocknet. Danach wird das so erhaltene Lyophylisat in einem Gemisch aus Methanol/Wasser unter Zusatz von Essigsäure gelöst. Der Wirkstoff kann aus der Lösung durch übliche chromatographische Methoden vorzugsweise Chromatographie an Sephadex LH 20® oder durch "reversed phase" Flüssigkeitshochdruckchromatographie gewonnen werden.

Der Wirkstoff wird als Leistungsförderer bei Tieren zur Förderung und Beschleunigung des Wachstums, der Milch-und Wollproduktion, sowie zur Verbesserung der Futterverwertung, der Fleischqualität und zur Verschiebung des Fleisch-Fett-Verhältnisses zugunsten von Fleisch eingesetzt. Besonders erwähnt werden soll ihre Eignung zur Verhinderung und Heilung der Schweinedysenterie und von Ketose. Der Wirkstoff wird bevorzugt bei Nutztieren verwendet. Zu den Nutztieren zählen Wiederkäuer wie z.B. Rinder, Schaft, Ziegen.

Der Wirkstoff wird unabhängig vom Geschlecht der Tiere während allen Leistungsphasen der Tiere eingesetzt. Bevorzugt wird der Wirkstoff während der intensiven Leistungsphase eingesetzt. Die intensive Leistungsphase dauert je nach Tierart von einem Monat bis zu 10 Jahren.

Die Menge des Wirkstoffs, die den Tieren zur Erreichung des gewünschten Effektes verabreicht wird, kann wegen der günstigen Eigenschaften des Wirkstoffs weitgehend variiert werden. Sie liegt vorzugsweise bei etwa 0,001 bis 50 mg/kg, insbesondere 0,01 bis 5 mg/kg Körpergewicht pro Tag. Die passende Menge des Wirkstoffs sowie die passende Dauer der Verabreichung hängen insbesondere von der Art, dem Alter, dem Geschlecht, der Leistungsphase, dem Gesundheitszustand und der Art der Haltung und Fütterung der Tiere ab und sind durch jeden Fachmann leicht zu ermitteln.

Der Wirkstoff wird den Tieren nach den üblichen Methoden verabreicht. Die Art der Verabreichung hängt insbesondere von der Art, dem Verhalten und dem Gesundheitszustand der Tiere ab.

Der Wirkstoff kann einmalig verabreicht werden. Der Wirkstoff kann aber auch während der ganzen oder während eines Teils der Leistungsphase temporär oder kontinuierlich verabreicht werden. Bei kontinuierlicher Verabreichung kann die Anwendung ein-oder mehrmals täglich in regelmäßigen oder unregelmäßigen Abständen erfolgen.

Die Verabreichung erfolgt oral in dafür geeigneten Formulierungen oder in reiner Form.

Der Wirkstoff kann in den Formulierungen allein oder in Mischung mit anderen leistungsfördernden Wirkstoffen, mineralischen Futtermitteln, Spurenelement-Verbindungen, Vitaminen, Nicht-Protein-Verbindungen, Farbstoffen, Antioxidantien, Aromastoffen, Emulgatoren, Fließhilfsstoffen, Konservierungs-und Preßhilfsstoffen vorliegen.

Antibiotika sind z.B. Tylosin und Virginiamycin.

Mineralische Futtermittel sind z.B. Dicalciumphosphat, Magnesiumoxid, Natriumchlorid.

Spurenelement-Verbindungen sind z.B. Eisenfumarat, Natriumjodid, Kobaltchlorid, Kupfersulfat, Zinkoxid.

Vitamine sind z.B. Vitamin A, Vitamin $D_3$, Vitamin E.

Nicht-Protein-Verbindungen sind z.B. Biuret, Harnstoff.

Farbstoffe sind z.B. Carotinoide wie Citranaxanthin, Zeaxanthin, Capsanthin.

Antioxidantien sind z.B. Ethoxyquin, Butylhydroxy-Toluol.

Aromastoffe sind z.B. Vanillin.

Emulgatoren sind z.B. Ester der Milchsäure, Lecithin. Fließhilfsstoffe sind z.B. Natriumstearat, Calciumstearat.

Konservierungsstoffe sind z.B. Zitronensäure, Propionsäure.

Preßhilfsstoffe sind z.B. Ligninsulfonate, Celluloseether.

Die Verabreichung des Wirkstoffs kann auch zusammen mit dem Futter und/oder dem Trinkwasser erfolgen.

Zum Futter zählen Einzelfuttermittel pflanzlicher Herkunft wie Heu, Rüben, Getreidenebenprodukte, Einzelfuttermittel tierischer Herkunft wie Fleisch, Fette, Milchprodukte, Knochenmehl, Fischprodukte, die Einzelfuttermittel wie Vitamine, Proteine, Aminosäuren z.B. DL-Methionin, Salze wie Kalk und Kochsalz. Zum Futter zählen auch Ergänzungs-, Fertig-und Mischfuttermittel. Diese enthalten Einzelfuttermittel in einer Zusammensetzung, die eine ausgewogene Ernährung hinsichtlich der Energie-und Proteinversorgung sowie der Versorgung mit Vitaminen, Mineralsalzen und Spurenelementen sicherstellt.

Die Konzentration des Wirkstoffs im Futter beträgt normalerweise etwa 1-500 ppm, bevorzugt 30-200 ppm.

Der Wirkstoff kann als solcher oder in Form von Prämixen oder Futterkonzentraten dem Futter zugesetzt werden.

Beispiel für die Zusammensetzung eines Futters für Rinder, das den erfindungsgemäßen Wirkstoff enthält: 69,95 % Futtergetreideschrot, 10 % gemahlene Maiskolben, 8 % Sojabohnenmehl, 5 % Luzernemehl, 5 % Melasse, 0,6 % Harnstoff, 0,5 % Calciumphosphat, 0,5 % Calciumcarbonat, 0,3 % Kochsalz und 0,15 % Prämix. Das Prämix enthält 70.000 i.E. Vitamin A, 70.000 i.E. Vitamin $D_3$, 100 mg Vitamin E, 50 mg Mangan, 30 mg Zink und 0,06 mg Kobalt. Der Wirkstoff wird dem Prämix in der erforderlichen Menge beigemischt.

Es ist nicht unbedingt erforderlich, gereinigten und isolierten Annomycin zu verwenden. Es ist auch möglich, das bei seiner Herstellung anfallende Gemisch oder sogar die anfallende Kulturbrühe oder das Mycelium ohne Reinigung, gegebenenfalls nach Trocknung, einzusetzen. Für viele Zwecke ist es auch ausreichend, Rohformen des erfindungsgemäßen Wirkstoffs und seiner Gemische ohne vorherige Feinreinigung einzusetzen.

Die Herstellung und biologische Wirkung der neuen erfindungsgemäßen Verbindung kann durch die folgenden Beispiele erläutert werden:

Beispiel A:

Einem Hammel, der pro Tag 650 g grob gemahlenes Schaf-Fertigfutter und 250 g Trockengrün Cobs erhielt, wurde Pansenflüssigkeit durch eine Pansenfistel entnommen. Das Fertigfutter wurde über einen Futterautomaten in 12 gleichen Portionen in zweistündlichem Abstand und die Cobs in 2 gleichen Portionen um 8.30 und 16.15 Uhr verabreicht. Die Pansenflüssigkeit wurde unmittelbar nach der Gewinnung folgender Behandlung unterworfen: 2,5 ml des Panseninokulums werden in einem mit Kohlendioxid begasten Reagenzröhrchen von 13 ml Volumen vorgelegt, das darüber hinaus folgende Zusätze enthielt:

100 mg fein gemahlenes Schaf-Fertigfutter

7,5 ml Pufferlösung

0,5 ml einer 5 % wäßrigen Ethanollösung mit bzw. ohne erfindungsgemäßen Wirkstoff.

Die Zusammensetzung der Pufferlösung, welche vor Anfang des Versuches mit Kohlendioxid gesättigt wurde, war wie folgt:

$Na_2HPO_4$    4,61 g per Liter Wasser
$NaHCO_3$    12,25 g per Liter Wasser
NaCl    0,59 g per Liter Wasser
KCl    0,71 g per Liter Wasser
$MgCl_2$    0,32 g per Liter Wasser
$CaCl_2$    0,13 g per Liter Wasser

Jedes Reagenzröhrchen wurde mit einem Bunsen-Stopfen verschlossen und bei 39°C bebrütet. Nach 1, 2, 4, 6 und 8 Stunden wurden die Ansätze von Hand geschüttelt. Nach 24-stündiger Bebrütung wurde 1,0 ml der Fermentationsflüssigkeit aus den Ansätzen entnommen und in ein Eppendorf-Gefäß pipettiert, das 0,2 ml 10 %ige Phosphorsäure (enthaltend 5,7 μmol 2-Methylvaleriansäure) enthielt. Die Proben wurden bei 11 000 g zentrifugiert und aus dem Überstand die flüchtigen Fettsäurekonzentrationen gaschromatographisch bestimmt.

Es wurde bei jedem Versuch das Verhältnis Essigsäure zu Propionsäure bestimmt. Der bei den Negativkontrollen erhaltene Wert wurde mit 100 angesetzt und die Abweichungen im Verhältnis dazu angegeben. Je mehr Propionsäure gebildet wurde, um so niedriger liegt das Verhältnis Essigsäure zu Propionsäure und um so kleiner wird die Verhältniszahl im Vergleich zur Kontrolle (niedrige Verhältniszahl = verringertes Essigsäure/Propionsäure-Verhältnis = verbesserte Futterverwertung).

Zusätzlich werden bei jedem Versuch die Konzentrationen der Gesamtfettsäuren im Vergleich zur Kontrolle (= 100) angegeben.

## Tabelle

| Menge (μg/Ansatz) | Essigsäure-Propionsäure-Verhältnis | Gesamtfettsäuren |
|---|---|---|
| Kontrolle | 100 | 100 |
| 20 | 88,2 | 104,7 |
| 100 | 60,9 | 103,8 |
| 250 | 55,5 | 105,2 |
| 1000 | 41,8 | 103,1 |

Beispiel 1

Herstellung des Impfgutes für die Vorkultur

150 ml sterile, in 1-Ltr.-Erlenmeyerkolben befindliche "Nährlösung A" CASO® der Firma Merck, Darmstadt der Zusammensetzung

| | |
|---|---|
| Pepton aus Casein | 15 g |
| Pepton aus Sojamehl | 5 g |
| D-Glucose | 2,5 g |
| NaCl | 5 g |
| Wasser | ad 1000 ml |

werden mit vegetativen Zellen des Streptomyceten-Stammes BS 572 beimpft und 3 Tage auf der Rundschüttelmaschine mit 280 Upm bei 28°C inkubiert. Die gewachsenen Kulturen dienen als Impfgut für weitere Fermentationsansätze.

Beispiel 2

Herstellung der Vorkultur

20 Liter Nährlösung der oben beschriebenen Zusammensetzung und 20 ml Entschäumer (SAG 5693, UnionCarbide) werden in einen Fermenter (30 l) mit Rührwerk und Belüftungseinrichtung abgefüllt, und bei 120°C sterilisiert. Nach Abkühlen der Lösung wird der Fermenter mit 300 ml der wie oben beschrieben gewonnenen Schüttelkulturen des Streptomyceten-Stammes BS 572 beimpft, mit 10 Liter steriler Luft pro Minute (0,5 VVm) bei 300 Umdrehungen des Rührwerks (Blattrührer) pro Minute belüftet und bei einer Temperatur von 28°C und einem Überlagerungsdruck von 0,5 bar fermentiert. Nach 2 tägiger Fermentation wird der Inhalt als Inokulum für einen Ansatz in einem 600 l Fermenter verwendet.

Beispiel 3 Tankfermentation

400 l der Nährlösung der folgenden Zusammensetzung

| | |
|---|---|
| Magermilchpulver | 10 g |
| Hefeautolysat | 1,5 g |
| Dextrin | 40 g |
| D-Glucose | 5 g |
| Antischaummittel (SAG 5693 Union Carbide) | 1 ml |
| ad | 1000 ml Wasser |

werden in einem 600 l Fermenter auf pH 7 eingestellt, 60 Minuten bei 121°C sterilisiert, auf 28°C abgekühlt und mit 20 l des gemäß Beispiel 2 erhaltenen Inoculums angeimpft. Es wird bei 28°C, einer Belüftungsrate von 180 l steriler Luft pro Minute (0,45 VVm), bei einem Überlagerungsdruck von 1,0 bar mit 100 Umdrehungen (Blattrührer) pro Minute fermentiert. Wenn nach 3-bis 4-tägiger Fermentation Annomycin gebildet worden ist, wird die Kultur geerntet.

Beispiel 4

a) Die gemäß Beispiel 3 gewonnene Kulturbrühe einer 400 l Fermentation wird mit ca. 200 l/Std. in einen Westfalia-Separator separiert. Der pH-Wert des Überstandes wird mit 6 N HCl auf 4,0 eingestellt und der Überstand über eine mit 50 l Lewapol OC 1031® (Bayer) beschickter Säule von 30 cm Durchmesser gegeben. Nacheinander wird mit 300 l entmineralisiertem Wasser 300 l (30 %igem) wäßrigem Methanol gewaschen und mit reinem Methanol desorbiert. Das Desorbat wird unter reduziertem Druck konzentriert, in ca. 20 l Wasser suspendiert und lyophylisiert, wobei 215 g des rohen erfindungsgemäßen Wirkstoffes erhalten werden. Der Wirkstoffgehalt beträgt 3 %.

b) Das Myzel wird mit 30 l Aceton 30 min gerührt und danach filtriert. Der Filterkuchen wird nochmals mit 20 l Aceton 30 min gerührt und erneut filtriert. Nun werden die gereinigten Filtrate unter reduziertem Druck auf ca. 15 l konzentriert und danach mit entmineralisiertem Wasser auf 130 l verdünnt. Diese Lösung wird nach Einstellen des pH-Wertes auf 4,0 mit 6 N HCl auf eine mit 20 l Lewapol OC 1031® beschickt Säule von 20 cm Durchmesser aufgegeben. Die Säule wird nacheinander mit 150 l entmineralisiertem Wasser, 150 l 30 %igem wäßrigem Methanol gewaschen und mit 140 l Methanol desorbiert. Nun wird unter reduziertem Druck kon zentriert in ca. 10 l Wasser suspendiert und gefriergetrocknet, wobei 96 g der rohen erfindungsgemäßen Verbindung erhalten werden. Der Wirkstoffgehalt beträgt 3 %.

## Beispiel 5

Reindarstellung der erfindungsgemäßen Verbindung durch Extraktion, Gelchromatographie und Reversed-Phase-Chromatographie

10 g des nach Beispiel 4a erhalten Rohproduktes werden mit 100 ml Wasser suspendiert und der pH-Wert mit 0,2 N KOH auf 8,0 eingestellt. Nun wird 20 min gerührt und danach zentrifugiert. Das Sediment wird in 50 ml Wasser suspendiert und gefriergetrocknet. Man erhält 1,5 g eines 12 %igen Rohproduktes.

2,7 g eines derartigen Rohproduktes werden in 30 ml einer 50 %igen wäßrigen methanolischen Lösung, die 50 ml mM an Essigsäure ist, gelöst und einer Gelfiltration auf Sephadex LH 20® unterworfen. Es wird eine Säule mit einem Durchmesser von 5 cm und einer Länge von 1m verwendet.

Die Fließrate beträgt 160 ml/Std. 20 ml Fraktionen werden genommen. Die nach Antibiographie gegen Staphylococcus aureus 1756 wirksamen Fraktionen werden vereinigt, unter reduziertem Druck konzentriert und gefriergetrocknet. Man erhält 314 mg des erfindungsgemäßen Antibiotikums mit einem Wirkstoffgehalt von 45 %.

180 mg des so erhaltenen Präparates werden in Portionen von 60 mg über eine präparative RP-18-Säule getrennt. Es wird eine Säule mit Durchmesser 16 mm und 25 cm Länge gefüllt mit Lichrosorb® RP 18 7 μm (Merck) verwendet. Als Fließmittel wird ein Gemisch von 0,1 $KH_2PO_4$ mit $H_3PO_4$ auf pH 2,1 eingestellt und Acetonitril im Verhältnis von Pufferlösung 6/Acetonitril 4 verwendet. Die Fließrate beträgt 15 ml/min. Es werden 16 ml Fraktionen gesammelt. Die Fraktionen werden dünnschichtchromatographisch analysiert und die mit Thymol/$H_2SO_4$ anfärbbaren, einheitlichen und biologisch aktiven Fraktionen vereinigt (180 ml) und unter reduziertem Druck konzentriert auf ca. 70 ml.

Sie werden auf eine Säule mit einem Durchmesser von 3 cm und 100 cm Länge gefüllt mit Sephadex LH 20® in 1 mM wäßrigen Essigsäure aufgetragen. Die Fließrate beträgt 70 ml/Std. 10 ml Fraktionen werden genommen. Die Fraktionen werden wie oben beschrieben analysiert, vereinigt und gefriergetrocknet. Ausbeute: 68 mg eines 95 %igen Produktes.

## Ansprüche

1. Organisch chemische Verbindung die durch die folgenden chemischen und physikalischen Parameter charakterisiert ist:

a) IR-KBr-Absorptionsspektrum zeigt bei folgenden Wellenlängen (in cm⁻¹) charakteristische Absorptionsbanden:

| | |
|---|---|
| 3388 | 1409 |
| 2936 | 1355 |
| 1731 | 1188 |
| 1639 | 1073 |
| 1536 | 972 |
| | 811 |
| | 652 |

b) 13C-Kernresonanzspektrum des Antibiotikums in deuteriertem Pyridin, gemäß Abb. 1, angegeben in Teilen pro Million.
Es wurde mit einer Feldstärke von 75,48 MHz an einer Lösung des Antibiotikums in deuteriertem Pyridin mit Tetramethylsilan als innerem Standard aufgenommen.

c) Das 13C-Kernresonanzspektrum des mit Acetanhydrid umgesetzten Antibiotikums in deuteriertem Pyridin gemäß Abb. 2, angegeben in Teilen pro Million.
Es wurde mit einer Feldstärke von 75,48 MHz an einer Lösung des Acetates in deuteriertem Pyridin mit Tetramethylsilan als innerem Standard aufgenommen.

d) Elementaranalyse (nach 2 Tagen Trocknen im Hochvakuum bei 30°C):
C 57,4 - 56,8
H 8,6 - 8,8
N 3,3 - 3,4

O 26,1 - 28,2

Es muß hier darauf hingewiesen werden, daß bei höhermolekularen Naturstoffen die Fehlerbreite der Elementaranalyse größer sein kann als allgemein üblich; daher ist eine genaue Bestimmung der Summenformel nicht möglich (R.B. Woodward, Angew. Chem. 69, 50-51 (1957)).

e) Die Verbindung läßt sich nach Chromatographie auf Kieselgel-Dünnschichtplatten mit Thymol/H$_2$SO$_4$ oder Cl$_2$-Tolidin Reagenz anfärben. Die Reagenzien wurden nach den üblichen Vorschriften (vgl. E. Stahl, Dünnschichtchromatographie, 2. Aufl., Springer Verlag, Berlin, Heidelberg, New York 1967) hergestellt.

f) Die antibakterielle Wirkung der Verbindung ist in Tabelle 1 wiedergegeben.

2. Verfahren zur Herstellung der organisch chemischen Verbindung gemäß Anspruch 1, dadurch gekennzeichnet, daß man Mikroorganismen der Familie Streptomycetaceae insbesondere der Gattung Streptomyces und vorzugsweise den Streptomyces-Stamm BS 572 (entsprechend DSM Nr. 3817) in einem Nährmedium welches assimilierbare Kohlenstoff-und Stickstoffquellen sowie Mineralsalze enthält, unter aeroben Bedingungen kultiviert und die gewünschte Verbindung isoliert.

3. Organisch-chemische Verbindung gemäß Anspruch 1, erhältlich nach dem Verfahren gemäß Anspruch 2.

4. Prämixe und/oder Futterzusatzmittel gekennzeichnet durch den Gehalt an der Verbindung die in den Ansprüchen 1-3 gekennzeichnet ist.

5. Verwendung der Verbindung, die in den Ansprüchen 1-3 gekennzeichnet ist bzw. der Prämixe und/oder Futterzusatzmittel gemäß Anspruch 4 zur Leistungsförderung bei Tieren.

6. Mikroorganismen der Familie Streptomycetaceae, insbesondere der Gattung Streptomyces, die bei einer Kultivierung in einem Kohlenstoff-und Stickstoffquellen sowie Mineralsalze enthaltendem Nährmedium die Verbindung, die in den Ansprüchen 1 und 2 gekennzeichnet ist, produzieren.

7. Streptomyces-Stamm BS 572 (gemäß DSM Nr. 3817) sowie seine Mutanten und Varianten.

8. Verwendung von Mikroorganismen gemäß den Ansprüchen 6 und 7 zur Herstellung der Verbindung, wie sie in den Ansprüchen 1 und 2 gekennzeichnet ist.

⊗ Solvens

FIG.1

FIG.2

⊗ Solvens

LeA 24714

0 259 751